# EUROPEAN PATENT APPLICATION

(11) **EP 4 563 003 A1**
(43) Date of publication of application: **04.06.2025**
(21) Application number: 24211333.0
(22) Date of filing: 07.11.2024
(51) Int. Cl.: A23B 7/10, A23L 19/20, A23L 27/10, A23L 27/12, A23L 27/50

(54) **KOREAN SAUCE MANUFACTURING METHOD**

(30) Priority: 29.11.2023 KR 20230169036
(71) Applicant: FoodBerry Co., Ltd., Andong-si Gyeongsangbuk-do (KR)
(72) Inventor: Ko, Yong Duck, Gyeongsangnam-do (KR); Jang, Jin Su, Gyeongsangbuk-do (KR); GWON, Yeong Rak, Gyeongsangbuk-do (KR)
(74) Representative: RGTH

(57) **Abstract**

The present invention is to provide a method of manufacturing a Korean sauce, the method including a purified water manufacturing step of obtaining raw water and purifying the raw water by causing the raw water to pass through a filtration filter and a reverse osmosis filtration membrane; a Korean sauce weighing step of weighing a received raw material and weighing an aged raw material; a lactic acid bacteria culture solution manufacturing step of performing main fermentation culture of lactic acid bacteria, homogenizing the lactic acid bacteria, and adding a predetermined amount thereof; a sauce mixture optimizing step of adding purified water and a liquid raw material which are measured in a predetermined mixing ratio, raising a temperature to a predetermined temperature, then adding a powder raw material, and stirring a resultant; a sterilization step of proceeding a sterilization operation in a predetermined temperature range and a predetermined time range by using an HTST sterilization device; a filtration step of filtering out a foreign substance and a filtrate that is out of a predetermined standard after the sterilization step; a filling step of filling a container with a predetermined amount; a cooling step of completely sealing an inside of the container with shrinkage vacuum pressure by a cooling process after the sterilization step; and a packaging step of attaching a label to which predetermined information is input to the container of the Korean sauce that has gone through the sterilization and cooling steps. According to an embodiment of the present invention, an object is to provide a manufacturing method of manufacturing a sauce with the original balanced flavor of food preferred by a consumer by a process of optimizing a mixing ratio to improve the flavor of Korean food.

## Description

### BACKGROUND OF THE INVENTION

### Field of the Invention

The present invention relates to a method of manufacturing a simple Korean sauce that can realize a Korean sauce flavor standardized and optimized so that anyone can easily and conveniently use the Korean sauce, and especially to suit the taste of Korean dish without any resistance to cooking beginners or foreigners.

In addition, the present invention relates to a manufacturing method that achieves an effect of enhancing the balanced flavor and long-term preservation of a Korean sauce by characteristically adding Schisandra chinensis syrup, undiluted juice of yuzu, black garlic concentrate, and plum juice that have gone through a natural aging process to a process of purifying water used in food manufacturing, homogenization and adding processes after culturing lactic acid bacteria, which have been shown to be effective in enhancing immunity, and a process of manufacturing a sweetener, an acidulant, a spice, and a preservative which are main raw materials by selecting optimal mixing conditions.

### Description of the Related Art

As a conventional Korean sauce, a sauce mainly made using a paste is used, which means that most of sauce ingredients are aged. However, in case of adding a flavor by using such an aged paste, it is difficult to differentiate a flavor due to a strong flavor that is caused by an aging process.

In addition, it is difficult to use the aged paste, especially overseas. If the flavor of the aged paste changes due to changes in the aging environment caused by differences in climate and varieties of raw materials, the flavor becomes different from a flavor when being domestically manufactured, making it difficult to maintain a uniform flavor and affecting the product quality.

In order to globalize Korean food that has flavors mostly based on a soy sauce and a red pepper paste, first, it is necessary to develop a sauce that can create uniform flavor even in overseas while also maintaining the unique flavor of a traditional sauce based on a soy sauce and a red pepper paste.

However, such traditional cooking liquids cannot be commonly used in various Korean dishes such as tang, jjigae, stir-fry, muchim, guk, and marinade, where main ingredients and cooking methods of dishes are different from each other. Therefore, depending on main ingredients and cooking methods of dishes, in order to prepare the desired dish, chefs had to separately prepare one or more cooking powders or cooking liquids and use an appropriate amount as needed.

In addition, in the related art, when cooking a specific dish, auxiliary ingredients that enhance the flavor and taste, such as green onions, garlic, and onions, have to be prepared in addition to main ingredients of a dish and be cooked together with the main ingredients at specific time, and seasoning has to be used at the completion stage of cooking. Since there were many types of main and auxiliary ingredients for the entire dish, there is a problem of giving up cooking if one does not have or lack of auxiliary ingredients.

### Citation List

### Patent Literature

Patent Literature 1: Korean Patent Registration No. 10-1435941 (August 25, 2014)
Patent Literature 2: Korean Patent Registration No. 10-1148354 (May 14, 2012)
Patent Literature 3: Korean Patent Registration No. 10-0956666 (April 29, 2010)
Patent Literature 4: Korean Patent Registration No. 10-2338839 (December 8, 2021)

### SUMMARY OF THE INVENTION

In order to solve the above-described problems of the present invention, the present invention relates to a method of manufacturing a simple Korean sauce that can realize a Korean sauce flavor standardized and optimized so that anyone can easily and conveniently use the Korean sauce, and especially to suit the taste of Korean dish without any resistance to cooking beginners or foreigners, and a device thereof.

In addition, the present invention relates to a manufacturing method that can achieve an effect of enhancing the balanced flavor and long-term preservation of a Korean sauce by characteristically adding Schisandra chinensis syrup, undiluted juice of yuzu, black garlic concentrate, and plum juice that have gone through a natural aging process to a process of purifying water used in food manufacturing, homogenization and adding processes after culturing lactic acid bacteria, which have been shown to be effective in enhancing immunity, and a process of manufacturing a sweetener, an acidulant, a spice, and a preservative which are main raw materials by selecting optimal mixing conditions.

As means for solving the above problems, provided is a method of manufacturing a Korean sauce, the method including: a purified water manufacturing step of obtaining raw water and purifying the raw water by causing the raw water to pass through a filtration filter and a reverse osmosis filtration membrane; a Korean sauce weighing step of weighing a received raw material and weighing an aged raw material; a lactic acid bacteria culture solution manufacturing step of performing main fermentation culture of lactic acid bacteria, homogenizing the lactic acid bacteria, and adding a predetermined amount thereof; a sauce mixture optimizing step of adding purified water and a liquid raw material which are measured in a predetermined mixing ratio, raising a temperature to a predetermined temperature, then adding a powder raw material, and stirring a resultant; a sterilization step of proceeding a sterilization operation in a predetermined temperature range and a predetermined time range by using an HTST sterilization device; a filtration step of filtering out a foreign substance and a filtrate that is out of a predetermined standard after the sterilization step; a filling step of filling a container with a predetermined amount; a cooling step of completely sealing an inside of the container with shrinkage vacuum pressure by a cooling process after the sterilization step; and a packaging step of attaching a label to which predetermined information is input to the container of the Korean sauce that has gone through the sterilization and cooling steps.

Meanwhile, in the purified water manufacturing step, the raw water is primarily filtered with a 25-micron filter and then secondarily filtered with a 5-micron filter, and then purified water is manufactured with a 1-micron reverse osmosis filtration membrane.

In the Korean sauce weighing step, the raw material includes a sweetener, an acidulant, a spice, and a preservative, and the aged raw material include Schisandra chinensis syrup, undiluted juice of yuzu, black garlic concentrate, and plum juice.

Next, in the sauce mixture optimizing step, the purified water and the liquid raw material that are measured in a predetermined mixing ratio are added, the temperature is raised to 50°C, then the powder raw material is added, and a stirring operation is proceeded.

Then, the sterilization step is proceeded for 20 ± 10 minutes under a temperature condition of 92 ± 5°C with respect to an inside of a food to be sterilized by using the HTST sterilization device.

### BRIEF DESCRIPTION OF THE DRAWINGS

FIG. 1 is a diagram illustrating a configuration of an apparatus that manufactures Korean sauce according to an embodiment of the present invention; and
FIG. 2 is a diagram illustrating a process and a sequence of manufacturing a Korean sauce according to the embodiment of the present invention.

### DETAILED DESCRIPTION OF THE PREFERRED EMBODIMENTS

In order to fully understand the present invention, operational advantages of the present invention, and the objectives achieved by implementing the present invention, reference should be made to the accompanying drawings illustrating preferred embodiments of the present invention and the contents described in the accompanying drawings.

Hereinafter, the present invention is described in detail by describing preferred embodiments of the present invention with reference to the accompanying drawings. However, the present invention may be implemented in many different forms and is not limited to the described embodiments. Also, in order to clearly describe the present invention, parts that are not relevant to the description are omitted, and like reference numerals in the drawings denote like members.

Hereinafter, various embodiments of the present document are described with reference to the accompanying drawings. However, the description is not intended to limit the technology described in the present document to specific embodiments and should be understood to include various modifications, equivalents, and/or alternatives to the embodiments of this document. In connection with the description of the drawings, like reference numerals in the drawings may be used for like components.

In the present document, expressions such as "include" or "may include" refer to the presence of the corresponding feature (e.g., a numerical value, a function, an operation, or a component such as parts) and do not exclude the presence of additional features.

Terms used in this document are merely used to describe specific embodiments and may not be intended to limit the scope of other embodiments. Singular expressions may include plural expressions, unless the context clearly indicates otherwise. Terms used herein, including technical or scientific terms, may have the same meaning as commonly understood by a person of ordinary skill in the technical field described in this document. Among the terms used in this document, terms defined in general dictionaries may be interpreted to have the same or similar meaning as the meaning the terms have in the context in the related technology and are not interpreted to have an ideal or excessively formal meaning, unless clearly defined in this document. In some cases, even if terms are defined in this document, the terms cannot be interpreted to exclude embodiments of this document.

It is obvious that various modifications can be made by those skilled in the art without departing from the gist of the present invention as claimed in the claims of the present invention, and these modifications should not be understood individually from the technical idea or prospect of the present invention.

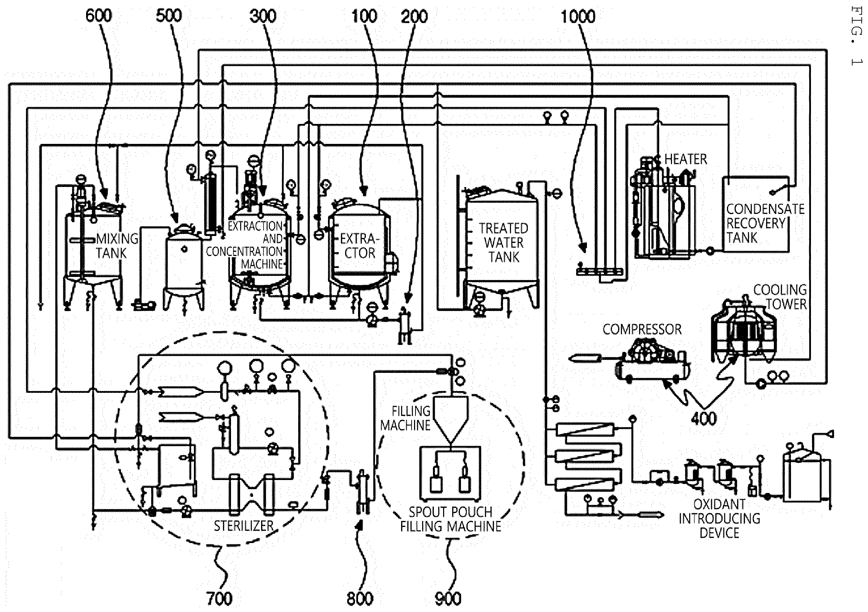

FIG. 1 is a diagram illustrating a configuration of an apparatus that manufactures Korean sauce according to an embodiment of the present invention.

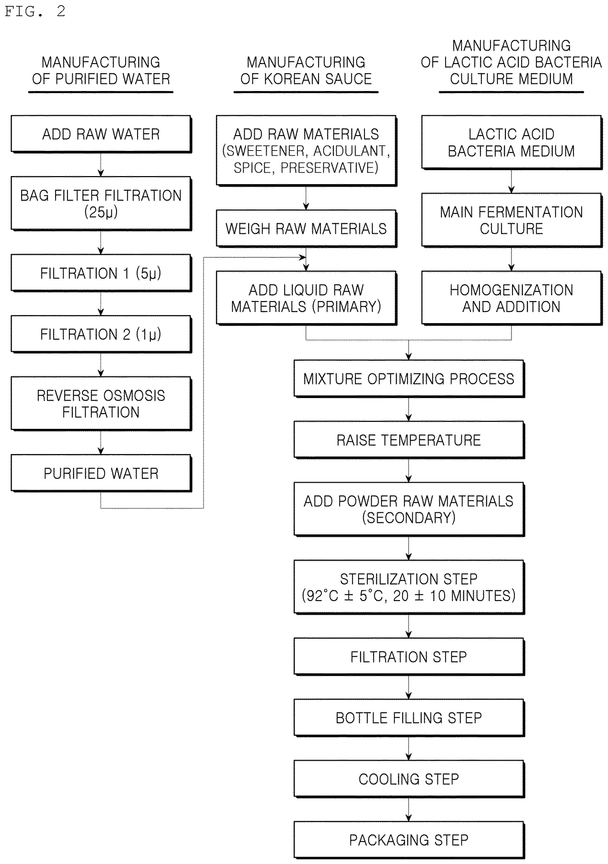

FIG. 2 is a diagram illustrating a process and a sequence of manufacturing a Korean sauce according to the embodiment of the present invention.

Referring to FIG. 1, the Korean sauce manufacturing device 10 according to the embodiment of the present invention is configured as follows.

Specifically, the Korean sauce manufacturing device 10 includes a raw water storage tank (not illustrated) that stores water supplied from a local water supply system, a reverse osmosis filtration device (not illustrated) that causes raw water stored in a raw water storage tank (not illustrated) to pass through a micro filter by using a raw water supply pump (not illustrated) so that turbidity, foreign substances, and the like included in the raw water are removed and that supplies the raw water to an osmosis membrane at high pressure by a high pressure pump so that fine foreign substances and ionic components remaining in the raw water are removed by the principle of reverse osmosis, a purified water storage tank (not illustrated) made of stainless steel that stores purified water, which is treated water that has passed through the reverse osmosis filtration device, a lactic acid bacteria culture tank 100 that injects the purified water stored in the purified water storage tank (not illustrated) and raw materials for Korean sauce and extracts the Korean sauce under multi-stage control of extraction target temperature and time, a raw material supply circulation pump 200 that circulates a black garlic raw material inside the lactic acid bacteria culture tank 100 up and down inside the lactic acid bacteria culture tank 100, a homomixer 300 for adjusting the final solid concentration from the Korean sauce extracted from the lactic acid bacteria culture tank 100, a primary filtration machine 500 for filtering the Korean sauce extracted from the lactic acid bacteria culture tank 100 with a 20 to 50-µ (micro) bag filter before transferring the Korean sauce to the homomixer 300, a cooling tower 400 for creating vacuum inside the homomixer 300 to collect the water separated from the solid content to the outside, cool the water, and store the water in a condensate recovery tank, a mixing tank 600 for mixing the solid content of the Korean sauce concentrated in the homomixer 300 with fragrance components and flavoring foods to mix a final product, a sterilizer that performs an instantaneous sterilization operation at an ultra-high temperature on the product mixed in the above mixing tank 600, a secondary filtration machine 800 that is equipped with a filtration filter that filters the product that has passed through the above sterilizer with 5µ (micro), and a filling machine 900 that individually packages the product that has passed through the above secondary filtration machine 800 into a specified capacity.

More specifically, the raw water storage tank (not illustrated) and the reverse osmosis filtration device (not illustrated) may be installed in the same space where the Korean sauce manufacturing device 10 is installed or may be installed outside separately.

The reverse osmosis filtration device (not illustrated) removes foreign substances and the like by causing the raw water that is transferred from the raw water storage tank (not illustrated) through a pipe by the raw water supply pump (not illustrated) to pass through a micro filter.

Then, the raw water is supplied to the osmosis membrane at high pressure by a high-pressure pump. In this manner, fine foreign substances and ionic components remaining in the raw water are removed from the raw water supplied to the osmosis membrane by the principle of reverse osmosis.

The reverse osmosis process is a primary filtration step in which the water is purified by passing through a 5-micro filter. Thereafter, the water is filtered again through a 1-micro filter in the second filtration step.

The purified water purified as described above is transferred by a predetermined required amount from the purified water storage tank (not illustrated) to the lactic acid bacteria culture tank 100 by driving a pump (not illustrated) through piping such as a pipe.

During the pretreatment process, the raw material for Korean sauce and purified water transferred from the purified water storage tank (not illustrated) are injected into the lactic acid bacteria culture tank 100 at a predetermined ratio.

Next, in the lactic acid bacteria culture tank 100, 1% to 3% of soybean (white bean) powder is first added to a typical lactic acid bacteria culture medium (MRS medium), a sterilization process is performed at 121°C for 15 minutes, the distributed public lactic acid bacteria strain *Lactobacillus plantarium M10* (KACC91481P) that is effective in enhancing immunity is then inoculated, and the homogenization process is performed through stirring the culture at 100 to 300 rpm at the culture temperature of 37 ± 2°C for the culture time of 18 ± 6 hours.

After the above process is completed, the immune-enhancing lactic acid bacteria culture is completed, and then a determined amount is transferred to the homomixer 300 through a transfer pump.

During the extraction process from the lactic acid bacteria culture tank 100, the raw materials for Korean sauce are separated into solid contents with certain nutrients and moisture at predetermined temperature and pressure.

Meanwhile, the lactic acid bacteria culture tank 100 is equipped with the raw material supply circulation pump 200. The raw material supply circulation pump is a pump that circulates black garlic and the purified water inside the lactic acid bacteria culture tank 100 up and down. Through the circulation action of the raw material supply circulation pump 200, heat is evenly delivered to the raw materials inside the lactic acid bacteria culture tank 100, so that the extraction operation proceeds smoothly and efficiently.

The solid content and moisture of the black garlic extracted as described above are filtered through the primary filtration machine 500.

The primary filtration machine 500 is equipped with a 100-micro filter. The filter is in the form of a bag filtration filter. Through the primary filtration, solid contents with large particles among of the solid contents separated through the lactic acid bacteria culture tank 100 are primarily filtered.

The solid contents less than 100 micro in size and the moisture filtered through the primary filtration machine 500 are transferred to the homomixer 300. Here, the extract of the primarily filtered black garlic is transferred to the homomixer 300 by a pump (not illustrated) through an extracted raw material supply pipe (not illustrated).

In the homomixer 300, the soybean fermented and extracted lactic acid bacteria solution manufactured in the lactic acid bacteria culture tank 100 and purified water and liquid raw materials measured in the mixing ratio are (primarily) added to homogenize the liquid raw materials, and the temperature is raised to 50°C to induce the food reaction between the source raw materials.

Next, when adding powder raw materials, in the case of fine powder, the powder raw materials are mixed evenly with powder with large particle size or powder with high water content before being added, and then equalization is performed by raising the rotation speed of the stirring blade together with the addition of the determined amount to the homomixer 300. The maintenance temperature of the homomixer of 85 ± 2°C is maintained for 10 minutes.

Then, after it is confirmed that the raw materials are transferred to the mixing tank 600 and that the fragrance components and flavor enhancing raw materials that are added to the raw materials of the sauce at the final step have the desired balanced sauce flavor in the mixing tank 600, the sterilization process which is the next process is performed.

The homomixer 300 performs a process of concentrating the Korean sauce transferred inside at vacuum decompression of 50 to 76 cmHg and a temperature of 55°C to 85°C for approximately 4 to 12 hours. Through the concentration process, a process of separating the Korean sauce into solid contents containing predetermined active components and moisture is performed.

In the homomixer, a cooler for condensing moisture evaporated from the raw material concentrated by applying steam heating and vacuum pressure and a vacuum pump for maintaining pressure lower than atmospheric pressure are driven to perform the moisture content separation operation in a short period of time.

The homomixer 300 is equipped with a stirrer to ensure that heat is evenly delivered to the raw materials concentrated inside. The stirrer is provided at one or more locations in the up-down direction inside the homomixer 300 and performs the action of stirring the raw materials stored inside while rotating in the left-right direction through the rotational force of the motor.

It is preferable that the stirrer rotates and performs stirring at a speed of 30 to 45 rpm. Through the stirring action by the stirrer, heat is evenly delivered to the Korean sauce which is the raw material, and the particles of the ingredients are evenly pulverized, so that the concentration process is performed smoothly.

Through the above process, the operation of adjusting the final solid concentration of the Korean sauce is proceeded.

Meanwhile, when the concentration process is completed in the homomixer 300, a process of removing the moisture from the homomixer 300 is performed.

The concentration process is a process of concentrating the raw material by applying high temperature heat and pressure to the inside of the homomixer 300. At this time, the moisture contained in the raw material undergoes a phase change to become high-temperature steam.

The homomixer 300 communicates with the cooling tower 400 through a pipe (not illustrated).

The cooling tower 400 includes a vacuum pump that causes the inside of the homomixer 300 to be a vacuum state.

Next, the process of performing the primary filtration is performed through the primary filtration machine 500.

The primary filtration machine 500 performs a process of performing filtration using a filter of 25 to 50 micro (µ). Through the above process, large particles are filtered out from the Korean sauce concentrated through the homomixer 300, and a process of making an extract with a fine density is performed.

The Korean sauce that has gone through primary filtration as described above is transferred to the mixing tank 600.

In the mixing tank 600, an operation of stirring the Korean sauce that has gone through the primary filtration once again using a stirrer is performed. This is to finally check the quality of the concentrated raw material liquid before packaging the concentrated raw material liquid and to improve the flavor of Korean sauce.

Next, the Korean sauce goes through a sterilization process using an instantaneous high-temperature sterilizer 700.

The instantaneous high-temperature sterilizer 700 performs a sterilization operation by heating the water stored in the condensate recovery tank with a heater and transferring the water through pipes in the form of high-temperature steam and water.

The instantaneous high-temperature sterilizer 700 performs a high-temperature short-time pasteurization process that performs sterilization at 130 to 135 degrees for one to five seconds.

The raw material that has gone through the instantaneous high-temperature short-time pasteurization process passes through the secondary filtration machine 800.

The secondary filtration machine 800 is equipped with a 5-micro (µ) filter. When filtration is performed through the secondary filtration machine 800, the Korean sauce is made into a final product with fine particles.

The Korean sauce that has completed the secondary filtration as described above is packaged into individual products with a predetermined volume through the filling machine 900.

The filling machine 900 performs a process of hot filling in which packaging is performed in the state of 92°C to 96°C.

With each process and configuration device as described above, a control unit 1000 controls each condition and required time.

The control unit 1000 controls whether each device should operate and operating time and conditions. The control of the control unit 1000 may be manually performed by an operator through each device and process or may be remotely and automatically performed by a program through a wired or wireless network.

In general, sauce refers to a material obtained by mixing paste, sugars, salt, vinegar, and the like with animal and vegetable raw materials or fermenting and aging the mixture. Preservatives are added to ensure long-term preservation properties, and chemical additives are added to enhance flavor and aroma, which deteriorates the original flavor of the food.

In contrast, the present invention provides a method for manufacturing a sauce having the original balanced flavor of food preferred by consumers by a process of optimizing a mixing ratio to enhance the flavor of Korean food by using the above-described manufacturing device.

Specifically, by using the Korean sauce manufacturing device, raw water is supplied and placed in a 20-ton stainless steel tank, sand or the like is then sedimented primarily, and the sediment is caused to pass through a housing filter system equipped with a 25-micron bag filter and to pass through a 5-micron fine precision filter and a 1-micron fine precision filter.

In the next process, purified water obtained by removing general microorganisms, algae, and the like and by passing through a reverse osmosis filtration membrane to filter out inorganic ions such as heavy metals in the water is manufactured to manufacture purified water to be used in sauce manufacturing.

More specifically, in the method for manufacturing Korean sauce, a purified water manufacturing step of obtaining raw water and causing the raw water to pass through a filtration filter and a reverse osmosis filtration membrane to purify the water is performed as described above.

Next, a Korean sauce weighing step of weighing the received raw materials for Korean sauce and weighing an aged raw materials to be added to the Korean sauce is performed.

After the weighing step is completed, a lactic acid bacteria culture solution manufacturing step of performing main fermentation culture of lactic acid bacteria, homogenizing the lactic acid bacteria, and adding a predetermined amount is performed.

Then, a sauce mixture optimizing step of adding purified water and a liquid raw material which are measured in a predetermined mixing ratio, raising a temperature to a predetermined temperature, adding a powder raw material, and stirring a resultant is performed by using the Korean sauce manufacturing device.

Then, a sterilization step of proceeding a sterilization operation in a predetermined temperature range and a predetermined time range by using a HTST sterilization device is performed. After the sterilization step, a filtration step of filtering out a foreign substance and a filtrate out of the predetermined standard is performed to manufacture the Korean sauce having predetermined uniform particles.

Thereafter, a filling step of filling a container with a predetermined amount is performed, and after the sterilization step, a cooling step of completely sealing the inside of the container using shrinkage vacuum pressure through a cooling process is performed.

Next, this manufacturing process is completed through a packaging step of attaching a label to which predetermined information is input to the container of the Korean sauce that has gone through the sterilization and cooling steps.

Meanwhile, in the purified water manufacturing step, the obtained raw water is primarily filtered using a 25-micron filter and secondarily filtered through a 5-micron filter, and then purified water is manufactured through a 1-micron reverse osmosis filtration membrane.

In addition, in the Korean sauce weighing step, the raw materials of the Korean sauce include a sweetener, an acidulant, a spice, and a preservative, and the aged raw materials of the Korean sauce include Schisandra chinensis syrup, undiluted juice of yuzu, black garlic concentrate, and plum juice.

In addition, the types and mixing ratios of raw materials and aged raw materials for Korean sauce are as shown in the table below.

Meanwhile, in the sauce mixture optimizing step, purified water and liquid raw materials measured at a predetermined mixing ratio are added, the temperature is raised to 50°C, powder raw materials are added, and a stirring operation is performed.

In addition, the sterilization step proceeds for 20 ± 10 minutes under a temperature condition of 92 ± 5°C based on the inside of the food to be sterilized by using an HTST sterilization device.

**[Table 1]**

| Mixture Mixing Ratio (%)_ Manufacturing Example | | | |
|---|---|---|---|
| Type | Manufacturing Example 1 | Comparative Example 1 | Comparative Example 2 |
| Lactic Acid Bacteria Culture Solution | 38.5 | 40.5 | 36.0 |
| Black Garlic Concentration Liquid | 19.2 | 17.4 | 21.2 |
| Plum Juice | 19.2 | 18.2 | 20.2 |
| Schisandra Chinensis Syrup | 13.5 | 14.5 | 12.0 |
| Undiluted Juice of Yuzu | 9.6 | 9.4 | 10.6 |

**[Table 2]**

| Ingredients of Korean Sauce and Manufacturing Mixing Ratio of Aged Liquid | | |
|---|---|---|
| Name of Manufacturing Raw Material | Addition Amount in Terms of Manufacturing 1 Kg | Mixing Ratio |
| Brewed Soy Sauce | 43 g | 38% to 48% |
| Sugar | 21 g | 17% to 25% |
| Purified Water | 19 g | 16% to 22% |
| Modified Starch | 0.8 g | 0.6% to 1.0% |
| Ground Garlic | 3 g | 2.7% to 3.3% |
| Ground Onion | 2 g | 1.8% to 2.2% |
| Ground Ginger | 0.5 g | 0.4% to 0.6% |
| Sodium L-glutamate | 1 g | 0.8% to 1.2% |
| Biofermented Nucleic Acid (I+G) | 0.1 g | 0.08% to 0.12% |
| Citric Acid | 0.2 g | 0.18% to 0.22% |
| Xanthan Gum | 0.1 g | 0.8% to 1.2% |
| Refined Salt | 4 g | 3.7% to 4.3% |
| Vitamin B1 Lauryl Sulfate | 0.15 g | 0.12% to 0.18% |
| Ethyl Paraoxybenzoate | 0.018 g | 0.015% to 0.021% |
| Lactic Acid Bacteria Culture Solution | 2 g | 0.04% to 0.06% |
| Black Garlic Concentrate | 1 g | 0.8% to 1.2% |
| Plum Juice | 1 g | 0.8% to 1.2% |
| Schisandra Chinensis Syrup | 0.7 g | 0.5% to 0.9% |
| Undiluted Juice of Yuzu | 0.5 g | 0.4% to 0.6% |

**[Table 3]**

| Manufacturing Ratio of Soybean-Derived Lactic Acid Bacteria Medium | |
|---|---|
| Name of Raw Material | Ratio (%) |
| Sucrose (White Sugar) | 2.0% |
| Soybean (Raw Soybean Powder) | 1.0% to 3.0% |
| Yeast Extract | 1.0% |
| Sodium Acetate | 0.5% |
| Dipotassium Phosphate | 0.2% |
| Ammonium Citrate | 0.2% |
| Tween80 | 0.2% |
| Magnesium sulfate | 0.01% |
| Manganese sulfate | 0.005% |
| Purified Water | 92.9% to 94.9% |

According to the present invention, it is possible to provide a method of manufacturing a simple Korean sauce that can realize a Korean sauce flavor standardized and optimized so that anyone can easily and conveniently use the Korean sauce, and especially to suit the taste of Korean dish without any resistance to cooking beginners or foreigners.

In addition, it is possible to achieve an effect of enhancing the balanced flavor and long-term preservation of a Korean sauce by characteristically adding Schisandra chinensis syrup, undiluted juice of yuzu, black garlic concentrate, and plum juice that have gone through a natural aging process to a process of purifying water used in food manufacturing, homogenization and adding processes after culturing lactic acid bacteria, which have been shown to be effective in enhancing immunity, and a process of manufacturing a sweetener, an acidulant, a spice, and a preservative which are main raw materials by selecting optimal mixing conditions.

While the present invention has been described with respect to the specific embodiments, it will be apparent to those skilled in the art that various changes and modifications may be made without departing from the spirit and scope of the invention as defined in the following claims.

## Claims

1. A method of manufacturing a Korean sauce, comprising:
a purified water manufacturing step of obtaining raw water and purifying the raw water by causing the raw water to pass through a filtration filter and a reverse osmosis filtration membrane;
a Korean sauce weighing step of weighing a received raw material and weighing an aged raw material;
a lactic acid bacteria culture solution manufacturing step of performing main fermentation culture of lactic acid bacteria, homogenizing the lactic acid bacteria, and adding a predetermined amount thereof;
a sauce mixture optimizing step of adding purified water and a liquid raw material which are measured in a predetermined mixing ratio, raising a temperature to a predetermined temperature, then adding a powder raw material, and stirring a resultant;
a sterilization step of proceeding a sterilization operation in a predetermined temperature range and a predetermined time range by using an HTST sterilization device;
a filtration step of filtering out a foreign substance and a filtrate that is out of a predetermined standard after the sterilization step;
a filling step of filling a container with a predetermined amount;
a cooling step of completely sealing an inside of the container with shrinkage vacuum pressure by a cooling process after the sterilization step; and
a packaging step of attaching a label to which predetermined information is input to the container of the Korean sauce that has gone through the sterilization and cooling steps.

2. The method of manufacturing a Korean sauce according to claim 1,
wherein in the purified water manufacturing step,
the raw water is primarily filtered with a 25-micron filter and then secondarily filtered with a 5-micron filter, and then purified water is manufactured with a 1-micron reverse osmosis filtration membrane.

3. The method of manufacturing a Korean sauce according to claim 1,
wherein in the Korean sauce weighing step,
the raw material includes a sweetener, an acidulant, a spice, and a preservative, and the aged raw material include Schisandra chinensis syrup, undiluted juice of yuzu, black garlic concentrate, and plum juice.

4. The method of manufacturing a Korean sauce according to claim 1,
wherein in the sauce mixture optimizing step,
the purified water and the liquid raw material that are measured in a predetermined mixing ratio are added, the temperature is raised to 50°C, then the powder raw material is added, and a stirring operation is proceeded.

5. The method of manufacturing a Korean sauce according to claim 1,
wherein the sterilization step is proceeded for 20 ± 10 minutes under a temperature condition of 92 ± 5°C with respect to an inside of a food to be sterilized by using the HTST sterilization device.
